(19) Europäisches Patentamt
European Patent Office
Office européen des brevets

(11) EP 1 246 633 B1

(12) **FASCICULE DE BREVET EUROPEEN**

(45) Date de publication et mention de la délivrance du brevet:
**21.07.2004 Bulletin 2004/30**

(21) Numéro de dépôt: **01903895.9**

(22) Date de dépôt: **09.01.2001**

(51) Int Cl.[7]: **A61K 35/78**, A61K 7/48, A23L 1/30

(86) Numéro de dépôt international:
**PCT/FR2001/000054**

(87) Numéro de publication internationale:
**WO 2001/051596 (19.07.2001 Gazette 2001/29)**

(54) **PROCEDE D'EXTRACTION DES INSAPONIFICABLES DES HUILES VEGETALES AU MOYEN DE CHLORO-1-BUTANE**

EXTRAKTIONSVERFAHREN DER UNVERSEIFBAREN AUS PFLANZENÖL MITTELS 1-CHLORBUTAN

METHOD FOR EXTRACTING UNSAPONIFIABLE MATTERS FROM VEGETABLE OILS USING CHLORO-1-BUTANE, COMPOSITION CONTAINING SAID UNSAPONIFIABLE MATTERS

(84) Etats contractants désignés:
**AT BE CH CY DE DK ES FI FR GB GR IE IT LI LU MC NL PT SE TR**

(30) Priorité: **12.01.2000 FR 0000333**

(43) Date de publication de la demande:
**09.10.2002 Bulletin 2002/41**

(73) Titulaire: **Laboratoires Expanscience**
**92419 Courbevoie Cedex (FR)**

(72) Inventeurs:
- **BARDET, Sandrine**
**F-16290 Asnières/Nouère (FR)**
- **LEGRAND, Jacques**
**F-61290 Neuilly-sur-Eure (FR)**
- **PICCIRILLI, Antoine**
**F-78000 Versailles (FR)**

(74) Mandataire: **Ahner, Francis et al**
**Cabinet Régimbeau**
**20, rue de Chazelles**
**75847 Paris cedex 17 (FR)**

(56) Documents cités:

| | |
|---|---|
| **FR-A- 2 678 632** | **FR-A- 2 762 512** |
| **GB-A- 1 142 804** | **US-A- 2 542 119** |

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention se rapporte à un procédé d'extraction des insaponifiables des huiles végétales ainsi qu'à des compositions pharmaceutiques et cosmétiques contenant les insaponifiables ainsi obtenus, et à l'utilisation de ces composés pour la fabrication d'un médicament, dans une méthode de traitement cosmétique et en tant qu'additif alimentaire.

Les composés insaponifiables constituent la fraction d'un corps gras qui, après action prolongée d'une base alcaline, reste insoluble dans l'eau et peut être extraite par un solvant organique.

Cinq grands groupes de substances sont présents dans la plupart des insaponifiables d'huiles végétales : hydrocarbures saturés ou insaturés, alcools aliphatiques ou terpéniques, stérols, tocophérols, les pigments caroténoïdes et xanthophiles.

Les composés insaponifiables sont recherchés pour leurs propriétés pharmacologiques, cosmétiques et nutritionnelles.

Les procédés usuels d'obtention des insaponifiables des huiles végétales comprennent une étape de saponification de la matière grasse et une extraction du produit cible (l'insaponifiable) par un solvant organique. Généralement, les solvants les plus communément utilisés sont les solvants des huiles, tels que les alcanes (hexane, heptane,...) et les solvants chlorés (dichloroéthane, trichloroéthane, tétrachlorure de carbone,...).

Parmi ces derniers, le dichloroéthane constitue le meilleur candidat, de par son rendement effectif d'extraction et sa sélectivité intrinsèque. Les insaponifiables sont en effet composés de nombreux constituants qu'il appartient d'extraire en totalité et avec un rendement maximal.

Cependant sur le plan industriel, la toxicité du solvant mis en oeuvre ainsi que sa stabilité chimique doivent être pris en compte. A ce titre, le dichloroéthane (DCE) présente deux inconvénients majeurs : le DCE est en effet classé parmi les solvants les plus toxiques, et présente par ailleurs un impact néfaste sur l'environnement. En outre en milieu basique (cas des solutions savonneuses de saponification), le DCE est partiellement dégradé.

Le problème posé par la présente invention est de choisir un solvant d'extraction qui soit moins toxique et chimiquement plus stable que le dichloroéthane et qui permette d'extraire les insaponifiables avec un rendement et une sélectivité au moins comparables aux rendements et sélectivités obtenus en utilisant le dichloroéthane, ce solvant pouvant aussi être mis en oeuvre dans un procédé de cristallisation.

La présente invention a ainsi pour objet un procédé d'extraction de la fraction insaponifiable contenue dans une huile végétale ou d'un co-produit de l'industrie du raffinage des huiles végétales tel que les échappées de désodorisation comprenant au moins :

A) une étape au cours de laquelle ladite huile est transformée en une solution hydro-alcoolique qui est de préférence une étape choisie dans le groupe constitué par les saponifications et les estérifications,
B) une étape d'extraction de la solution hydro-alcoolique au cours de laquelle la fraction grasse est séparée de la fraction insaponifiable choisie dans le groupe constitué par les extractions liquide-liquide et les distillations,
C) éventuellement, une étape de purification de l'insaponifiable choisie dans le groupe constitué par les cristallisations et les extractions liquide-liquide, tel qu'au moins une étape parmi les extractions liquide-liquide de l'étape B, les cristallisations de l'étape C ou les extractions liquide-liquide de l'étape C est effectuée en utilisant le chloro-1-butane.

Le procédé selon la présente invention permet d'extraire une fraction insaponifiable contenue dans une huile végétale, il permet aussi d'extraire un co-produit de l'industrie du raffinage des huiles végétales, comme par exemple les échappées de désodorisation, encore appelées déodistillats, produites au cours du raffinage des huiles végétales.

Les acides gras et les glycérides partiels présents dans les déodistillats peuvent en effet être saponifiés ou estérifiés par un alcool léger, dans le but de séparer la fraction grasse de la fraction insaponifiable, soit par extraction liquide-liquide ou distillation sous vide. Enfin, la purification de l'insaponifiable ou des fractions actives séparées, le plus souvent les tocophérols (vitamine E) et les stérols, met en jeu notamment des étapes de cristallisation dans un solvant organique ou d'extraction liquide-liquide.

La présente invention a encore pour objet un procédé d'extraction de la fraction insaponifiable contenue dans une huile végétale comprenant au moins :

A) une étape de saponification par laquelle ladite huile est transformée en une solution hydro-alcoolique,
B) une étape d'extraction de la solution hydro-alcoolique par un solvant organique tel que le solvant organique est le chloro-1-butane, de préférence l'huile végétale traitée est une huile d'avocat ou de soja.

Plus particulièrement, le procédé d'extraction de la fraction insaponifiable d'une huile d'avocat selon la présente invention est tel que l'on effectue une extraction à contre-courant de la solution hydro-alcoolique au moyen de

chloro-1-butane, le rapport (volume/volume) v/v chloro-1-butane / solution hydro-alcoolique étant compris entre 0,5 et 5, de préférence compris entre 0,9 et 1,2 et de manière encore plus préférée d'environ 1.

Plus particulièrement, le procédé d'extraction de la fraction insaponifiable d'une huile de soja selon la présente invention est tel que l'on effectue une extraction à contre-courant de la solution hydro-alcoolique au moyen de chloro-1-butane, le rapport (volume/volume) v/v chloro-1-butane / solution hydro-alcoolique étant compris entre 0,5 et 5 de préférence compris entre 0,9 et 1,5 et de manière encore plus préférée d'environ 1,3.

La présente invention a également pour objet un procédé d'extraction de la fraction insaponifiable d'un co-produit de l'industrie du raffinage d'une huile végétale, tel que ce co-produit est un déodistillat d'une huile végétale, ledit procédé comprenant au moins :

- une étape de saponification par laquelle le déodistillat est transformée en une solution hydro-alcoolique,
- une étape d'extraction à contre-courant de la solution hydro-alcoolique au moyen de chloro-1-butane,
- une étape de cristallisation des stérols et/ou des alcools triterpéniques,
- une étape d'isolation d'un composé actif tel que les tocophérols, les tocotriénols, le squalène, les carotènes, la sésamine, la sésamoline, étape choisie dans le groupe formé par les extractions de préférence au moyen de chloro-1-butane, et les distillations.

La présente invention a encore pour objet la fraction insaponifiable obtenue par le procédé d'extraction selon la présente invention.

La comparaison des teneurs en insaponifiables de différentes huiles végétales : soja, coton, noix de coco, olive et avocat, montre que l'huile d'avocat obtenue par extraction suivant divers procédés connus comprend un taux particulièrement important d'insaponifiables. Typiquement, les teneurs obtenues s'échelonnent de 2 à 7% d'insaponifiables dans l'huile d'avocat contre 0,5% dans l'huile de coco, 1% dans l'huile de soja, 1% dans l'huile d'olive. L'insaponifiable d'avocat peut être préparé par extraction à partir de l'huile d'avocat.

Le procédé d'extraction des insaponifiables d'une huile d'avocat peut être effectué de la façon suivante.

Selon une méthode connue de l'homme du métier :

- soit on presse la pulpe fraîche en présence d'un tiers corps fibreux absorbant l'eau tel que la parche de café dans une presse à cage, puis on sépare l'émulsion d'huile et d'eau obtenue par décantation et/ou centrifugation ;
- soit on broie la pulpe fraîche et on la met en contact avec un solvant organique adapté (par exemple un mélange méthanol-chloroforme) puis on récupère l'huile par évaporation du solvant.

Plusieurs procédés ont été décrits dans l'art antérieur pour extraire la fraction insaponifiable d'une huile végétale.

On peut citer en particulier le procédé de préparation d'insaponifiable d'huile d'avocat tel que décrit et revendiqué dans le brevet FR-2 678 632 au nom des Laboratoires Pharmascience. Ce procédé permet d'obtenir un insaponifiable d'avocat riche en fraction H en comparaison aux procédés classiques de préparation d'insaponifiable d'avocat.

Ainsi, l'insaponifiable d'huile d'avocat utilisé selon l'invention peut être obtenu à partir du fruit frais mais, de préférence, l'insaponifiable d'avocat est préparé à partir du fruit préalablement traité thermiquement, avant l'extraction de l'huile et la saponification, comme décrit dans le brevet FR-2 678 632. Ce traitement thermique consiste en un séchage contrôlé du fruit, frais de préférence, pendant au moins quatre heures, avantageusement au moins 10 heures, de préférence entre environ 24 et environ 48 heures, à une température de préférence d'au moins environ 80 °C et de préférence comprise entre environ 80 et environ 120 °C.

On peut également citer le procédé de préparation d'insaponifiable d'huile de soja, obtenu à partir d'un concentrat d'insaponifiable d'huile de soja. Ledit concentrat d'insaponifiable est préparé par distillation moléculaire selon un procédé tel que décrit pour l'huile de lupin dans la demande de brevet FR-2 762 512, mais adapté à l'huile de soja. Dans ce procédé, l'huile de soja est distillée dans un distillateur moléculaire de type centrifuge ou à film raclé, à une température comprise entre environ 210 et 250° C et sous un vide poussé, compris entre 0,01 et 0,001 millimètres de mercure (soit 0,13 à 1,3 Pa). Le distillat obtenu présente une teneur en insaponifiable comprise entre 5 et 40% en poids et constitue donc un concentrat d'insaponifiable d'huile de soja.

Le concentrat est ensuite saponifié par une base telle que la potasse ou la soude en milieu alcoolique, de préférence éthanolique. Puis il est soumis à une ou plusieurs extractions par le chloro-1-butane.

La solution d'extraction obtenue est de préférence ensuite centrifugée, filtrée puis lavée à l'eau pour éliminer les traces résiduelles d'alcalinité.

Le solvant d'extraction est évaporé soigneusement pour récupérer l'insaponifiable. On peut également bien entendu prévoir des opérations supplémentaires connues de l'homme du métier, telle qu'une étape de désodorisation.

Enfin, avant sa saponification, l'huile peut être préalablement enrichie en insaponifiable en séparant une

majorité des constituants de l'insaponifiable que l'on récupère dans un concentrat. Différentes méthodes peuvent être utilisées : cristallisation par le froid, extraction liquide-liquide, distillation moléculaire.

La concentration préalable de l'huile en insaponifiable permet de diminuer la consommation d'huile pendant la saponification. La distillation moléculaire est particulièrement préférée, étant réalisée de préférence à une température comprise entre environ 180 et environ 230 C° en maintenant une pression comprise entre $10^{-3}$ et $10^{-2}$ mmHg et de préférence de l'ordre de $10^{-3}$ mmHg. La concentration en insaponifiables du distillat peut atteindre 60%.

La présente invention a encore pour objet une composition cosmétique comprenant au moins une fraction insaponifiable contenue dans une huile végétale et un excipient cosmétiquement acceptable, une composition pharmaceutique ou composition à titre de médicament comprenant au moins une fraction insaponifiable contenue dans une huile végétale et un excipient pharmaceutiquement acceptable et une composition alimentaire comprenant au moins une fraction insaponifiable contenue dans une huile végétale.

La présente invention vise encore un procédé de traitement cosmétique tel que l'on applique topiquement la composition cosmétique selon l'invention et aussi l'utilisation d'un insaponifiable d'une huile végétale obtenu selon la présente invention pour la fabrication d'un médicament.

A titre d'exemples illustrant la présente invention, deux séries d'expérimentations ont été successivement effectuées.

**EXEMPLE 1 : Première série d'extractions (en ampoule à décanter).**

Avant extraction de l'insaponifiable, on effectue au cours d'une première étape, une saponification du concentrat de l'huile considérée (avocat ou soja), ce concentrat étant préalablement obtenu par distillation moléculaire de l'huile.

Après saponification, on obtient une solution hydro-alcoolique, appelée SHA, contenant l'insaponifiable en solution. Cet insaponifiable est alors extrait par un solvant organique : le DCE choisi comme référence et le chloro-1-butane (C1B).

Dans un ballon de 100 ml équipé d'un réfrigérant, on pèse précisément la masse de concentrat à saponifier (plus éventuellement l'huile de dilution pour l'avocat). On ajoute alors l'alcool éthylique, la potasse, quelques grains de pierre ponce, et on porte à reflux pendant 4 heures. Après refroidissement, on effectue une dilution du milieu réactionnel par de l'eau déminéralisée.

L'extraction est mise en oeuvre dans une ampoule à décanter. Les phases organiques sont alors rassemblées puis lavées à l'eau de ville jusqu'à neutralité (test à la phénolphtaléine).

La phase solvant obtenue est ensuite séchée sur du sulfate de sodium anhydre (3g). Après filtration, le solvant est évaporé à l'évaporateur rotatif. L'insaponifiable récupéré est enfin pesé puis stocké sous azote, avant analyse en CPG (chromatographie phase gazeuse) et HPLC (chromatographie phase liquide haute performance).

***Résultats***

Après optimisation des conditions d'extraction (tableaux 1 et 2), les résultats obtenus sont rassemblés dans les tableaux 3 et 4.

Tableau 1 :

| ***Conditions de saponification et d'extraction des concentrats d'avocat*** | | |
|---|---|---|
| | DCE | C1B |
| Concentrat d'avocat (g) | 10 | 10 |
| Potasse (g) | 5 | 5 |
| Alcool (ml) | 24 | 44 |
| Eau de dilution (ml) | 35 | 30 |
| Solvant d'extraction | 5 X 35 ml | 7 X 50 ml |
| Lavage | 5 X 100 ml | 6 X 50 ml |

Tableau 2 :

| *Condition de saponification et d'extraction des concentrats de soja.* | | |
|---|---|---|
| | DCE | C1B |
| Concentrat Soja(g) | 10 | 10 |
| Potasse (g) | 4 | 4 |
| Alcool (ml) | 24 | 44 |
| Eau de dilution (ml) | 35 | 25 |
| Solvant d'extraction | 5 X 35 ml | 6 X 50 ml |
| Lavage | 5 X 100 ml | 6 X 50 ml |

Tableau 3 :

| ***Analyses et rendements d'extraction des insaponifiables d'avocat.*** | | |
|---|---|---|
| **Analyse insaponifiable (%)(1)** | **DCE** | **C1B** |
| Composés furaniques | 70,0 | 67,2 |
| Alcool gras polyhydroxylés | 15,7 | 16,6 |
| Hydrocarbures | 1,0 | 0,5 |
| Squalène | 1,5 | 1,3 |
| Tocophérols | Traces | Traces |
| Stérols | 4,7 | 3,9 |
| Autres | 7,1 | 10,5 |
| **Rendements d'extraction (%) (1)** | **41,7** | 46,3 |

(1) pourcentages ramenés au concentrat qui est la composition disponible avant saponification.

Tableau 4 :

| ***Analyses et rendements d'extractions des insaponifiables de soja*** | | |
|---|---|---|
| **Analyse insaponifiable(%)(1)** | **DCE** | **C1B** |
| Hydrocarbures | 0,8 | 0,7 |
| Squalène | 9,8 | 11,6 |
| Tocophérols | 28,4 | 21,4 |
| Stérols | 60,4 | 66,3 |
| **Rendements d'extraction (%) (1)** | **36,4** | **34,8** |

(1) pourcentages ramenés au concentrat qui est la composition disponible avant saponification.

Ces résultats (tableau 3) montrent que les insaponifiables d'avocat sont extraits en présence du chloro-1-butane avec des rendements supérieurs à ceux obtenus avec du DCE. Par ailleurs, la composition des insaponifiables est proche de la référence (insaponifiables extraits par le DCE).

Les résultats du tableau 4 montrent qu'avec le chloro-1-butane, les insaponifiables de soja sont extraits avec un rendement très légèrement inférieur au DCE et que la composition des insaponifiables est très proche de la référence (insaponifiable extrait de DCE).

En conclusion, le chloro-1-butane s'avère être un excellent candidat de substitution aux solvants classiques des insaponifiables.

**EXEMPLE II : Deuxième série d'extractions.**

Ces essais ont été réalisés sur une batterie micropilote de mélangeurs-décanteurs de marque Robatel, dont le fonctionnement permet une extrapolation des résultats à un extracteur industriel de type batterie de mélangeur-décanteur ou de type colonne pulsée à contre-courant.

La solution à extraire est une solution hydro-alcoolique (SHA), issue d'une réaction de saponification. En effet, avant d'effectuer l'extraction liquide-liquide de l'insaponifiable, le procédé d'obtention de ce même insaponifiable comprend deux étapes préalables. La première consiste à concentrer la fraction insaponifiable par distillation moléculaire de l'huile brute ou raffinée. La seconde concerne la saponification du concentrat d'insaponifiable obtenu, en présence de potasse en milieu alcoolique. En fin de réaction, le mélange est dilué par de l'eau déminéralisée puis envoyé dans une colonne pulsée afin d'être soumis à l'action extractive d'un solvant organique, propre à réaliser l'extraction liquide-liquide de l'insaponifiable.

**1) Préparation de la solution hydro-alcoolique à extraire (SHA)**

Dans un réacteur double enveloppe (chauffage vapeur) de type Grignard de 100 litres, équipé d'un agitateur mécanique et surmonté d'un réfrigérant, on introduit successivement les quantités requises de potasse aqueuse, d'alcool et de corps gras à saponifier. Le mélange est porté sous agitation constante à la température de reflux de l'éthanol (70-80°C) puis maintenu à cette température pendant 3,5 heures. Après réaction, le mélange est refroidi à 30°C puis dilué par ajout de la quantité requise d'eau déminéralisée de dilution. La solution hydro-alcoolique obtenue est alors envoyée dans la batterie de mélangeur-décanteur afin de subir l'étape d'extraction liquide-liquide.

Tableau 5 :

| ***compositions des milieux de saponification*** | | |
|---|---|---|
| **REACTIFS** | **Saponification d'un Concentrat d'avocat(1)** | **Saponification d'un concentrat de soja (1)** |
| Concentrat* | 10 | 10 |
| Alcool éthylique * | 25 | 16 |
| Potasse aqueuse à 50% * | 5 | 4 |
| Eau de dilution finale * | 48 | 30 |

(1 ) :concentrat obtenu par distillation moléculaire de l'huile de départ
* :en kilogramme

**2) Extraction liquide-liquide des insaponifiables**

L'extraction liquide-liquide des insaponifiables d'avocat et de soja est réalisée dans une batterie de mélangeur-décanteur de type Robatel. L'extracteur est alimenté par la solution hydro-alcoolique d'avocat ou de soja, SHA, puis à contre-courant par le solvant utilisé (dichloroéthane ou chloro-1-butane). Pour contrôler la mise en régime stationnaire de l'extracteur, les débits sont relevés toutes les 15 minutes. Lorsque la mise en régime est atteinte, on procède après 60 minutes de fonctionnement à des prélèvements de chaque phase (extrait et raffinat). L'analyse des phases prélevées est réalisée en continu.

La quantité d'insaponifiable extrait par rapport à la solution hydro-alcoolique obtenue après saponification est déterminée de la façon suivante :

$$\% \text{ Insaponifiable extrait} = 100 \times (D_{Ex} \times X_{Ex})/(D_{SHA} \times X_{SHA})$$

Avec:

- $\mathbf{D_{Ex}}$ : débit massique de l'extrait
- $\mathbf{D_{SHA}}$ : débit massique de la solution hydro-alcoolique
- $\mathbf{X_{Ex}}$ : titre massique de l'extrait en %
- $\mathbf{x_{SHA}}$ : titre massique de la solution hydro-alcoolique en %

**3) Résultats**

**3.1 Extraction des insaponifiables d'avocat**

***Extraction comparative au DCE (Dichloroéthane) et au chloro-1-butane (C1B)***

***Tableau 6 : Comparaison des propriétés extractives du Dichloroéthane (DCE) et du Chloro-1-butane (C1B)***

| SOLVANT | RAPPORT SOLVANT/SHA | INSAPONIFIABLE EXTRAIT (%) |
|---|---|---|
| DCE | 1,0 | 95,0 |
| C1B | 1,0 | 97,7 |

| SOLVANT | COMPOSITION DE L'INSAPONIFIABLE EXTRAIT | | | | | |
|---|---|---|---|---|---|---|
| | Fraction H | Fraction I | Stérols | Squalène | Acides gras | Autres |
| DCE | 67,6 | 8,0 | 4,4 | 1,3 | 0,4 | 18,3 |
| C1B | 75,8 | 1,9 | 4,1 | 1,4 | 0,4 | 16,4 |

La fraction H représente les furanes de l'avocat, et plus particulièrement, les composés de formule :

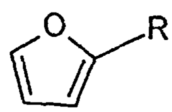

dans laquelle R est une chaîne linéaire hydrocarbonée en $C_{11}$-$C_{19}$ de préférence $C_{13}$-$C_{17}$ saturée ou comprenant une ou plusieurs insaturations éthyléniques ou acétyléniques,

et la fraction I représente les alcools gras polyhydroxylés de l'avocat et plus particulièrement, par « alcools gras polyhydroxylés d'avocat », on entend polyols sous forme d'une chaîne principale linéaire hydrocarbonée en $C_{17}$-$C_{21}$ saturée ou comprenant une ou plusieurs insaturations éthyléniques ou acétyléniques, et comprenant au moins deux groupes hydroxyles majoritairement situés sur une partie de la chaîne principale, l'autre partie de cette chaîne principale constituant ainsi la chaîne « grasse » (partie hydrophobe) du polyol.

Ces résultats montrent que :

- Le chloro-1-butane s'avère un excellent solvant d'extraction des insaponifiables d'avocat.
- La quantité d'insaponifiable extrait est au moins égale, voire supérieure à celle du dichloroéthane, reconnu comme un excellent solvant d'extraction des insaponifiables.
- Sur le plan de leur composition, les insaponifiables extraits sont très proches. Toutefois, le chloro-1-butane est plus sélectif en fraction H, au détriment de la fraction I (alcools gras polyhydroxylés de l'avocat).

*Influence du rapport solvant/ SHA*

Tableau 9 :

| *Influence du rapport Chloro-1-butane/Solution hydro-alcoolique (SHA)* | | | | |
|---|---|---|---|---|
| **Rapport massique Chloro-1- butane/ SHA** | DCE/SHA =1 (1) | 0,8 | 0,9 | 1,2 |
| **Insaponifiable extrait (%)** | 95,5 | 88,6 | 94,8 | 98,7 |
| **Fraction H (%)** | 67,6 | 74,4 | 72,4 | 71,3 |
| **Fraction I (%)** | 8,0 | 1,2 | 2,0 | 2,1 |
| **Stérols (%)** | 4,4 | 3,1 | 3,7 | 3,9 |
| **Acides gras (%)** | 0,4 | 0,4 | 0,3 | 0,5 |
| **Autres (%)** | 19,6 | 20,9 | 21,6 | 22,2 |

(1) Dichloroéthane: solvant de référence

- La quantité d'insaponifiable extrait tend à augmenter avec le rapport chlorobutane/SHA.
- Le rapport optimal se situe entre 0,9 et 1,2.

**3.2 Extraction des insaponifiables de soja**

L'extraction des insaponifiables de soja nécessite le maintien de la solution hydro-alcoolique à une température comprise entre 35 C° et 40 C°.

*Extraction comparative au DCE (Dichloroéthane) et au chloro-1-butane (C1B)*

***Tableau 10 : Comparaison des propriétés extractives du Dichloroéthane (DCE) et du Chlorobutane (C1B)***

| SOLVANT | RAPPORT SOLVANT/SHA | INSAPONIFIABLE EXTRAIT (%) |
|---|---|---|
| DCE | 1,0 | 90,7 |
| C1B | 1,3 | 97,7 |

| SOLVANT | COMPOSITION DE L'INSAPONIFIABLE EXTRAIT | | | |
|---|---|---|---|---|
| | Stérols | Tocophérols | Hydrocarbures | Squalène |
| DCE | 65,5 | 20 ,8 | 1,3 | 12,4 |
| C1B | 62,0 | 22,5 | 1,0 | 14,5 |

Les résultats montrent que:

- Le chloro-1-butane s'avère un excellent solvant d'extraction des insaponifiables de soja.
- La quantité d'insaponifiable extrait est supérieure à celle du dichloroéthane, reconnu comme un excellent solvant d'extraction des insaponifiables.
- Sur le plan de leur composition, les insaponifiables extraits sont très proches. Ce solvant est donc parfaitement

adapté à l'extraction des insaponifiables d'huiles végétales classiques.

**EXEMPLE III : Extraction des fractions insaponifiables valorisables d'un distillat d'huile de soja.**

**Exemple III-1.**

Dans un réacteur de type Grignard, 100 kg de déodistillat d'huile de soja sont saponifiés à reflux en présence de 40 kg de potasse aqueuse (à 50%) et de 200 litres d'alcool éthylique. Après saponification, le mélange réactionnel est dilué par ajout de 300 kg d'eau déminéralisée puis envoyé dans une colonne d'extraction pulsée, à contre-courant. Le solvant mis en oeuvre est le chloro-1-butane (rapport solvant/solution hydro-alcoolique =1). Après extraction, la phase organique est lavée dans une colonne de lavage alimentée en continu par de l'eau déminéralisée. Le chloro-1-butane, solvant d'extraction, est enfin séparé de l'insaponifiable brut dans un évaporateur de type flot tombant.

Après évaporation, l'insaponifiable brut obtenu est dissout à chaud dans 65 litres d'éthanol. Le mélange est alors refroidi sous agitation mécanique lente de 70°C à 15°C et maintenu 2 heures et demie à cette température. La cristallisation des stérols s'amorce à 45°C. Après essorage rapide sur büchner, on effectue une série de lavages à l'éthanol à 50%, puis à l'eau déminéralisée des stérols cristallisés. Le gâteau de filtration, constitué des stérols extraits, est enfin séché dans une étuve ventilée à 80°C pendant 12 heures. On obtient ainsi 9,7 kg de stérols dont la pureté est proche de 98%.

La phase éthanolique récupérée est ensuite évaporée. L'insaponifiable obtenu, partiellement destérolé, subit alors une étape de distillation moléculaire à 120°C, sous un vide de $10^{-3}$ mm de mercure. On récupère 11,8 kg de distillat (fraction légère) composé à 90% d'hydrocarbures. Le résidu (fraction lourde) présente une teneur en tocophérols proche de 50%.

**Exemple III-2.**

100 kg de déodistillat d'huile de soja sont distillés à 130°C et sous un vide de $10^{-3}$ mm de mercure. On récupère une fraction légère (41,5 kg) composée essentiellement d'hydrocarbures et d'acides gras ainsi qu'une fraction lourde (58,5 kg), riche en stérols et tocophérols. Cette dernière est alors saponifiée de façon identique à l'exemple 1, en présence de 90 litres d'éthanol et 15 kg de potasse aqueuse à 50%.

Le mélange obtenu est alors refroidi sous agitation mécanique lente de 80°C à 15°C et maintenu à cette température pendant 1 heure. La cristallisation des stérols s'amorce à 45°C. On effectue alors un essorage rapide sur büchner, puis une série de lavages à l'éthanol à 50% ainsi qu'à l'eau déminéralisée. Le gâteau de filtration, constitué des stérols extraits, est enfin séché dans une étuve ventilée à 80°C pendant 12 heures. On obtient ainsi 9,1 kg de stérols dont la pureté est supérieure à 95%.

Le filtrat récupéré est alors dilué par 60 litres d'eau déminéralisée puis envoyé dans une colonne d'extraction pulsée, à contre-courant. Le solvant mis en oeuvre est le choro-1-butane (rapport solvant/solution hydro-alcoolique=1). Après extraction, la phase organique est lavée dans une colonne de lavage alimentée en continu par de l'eau déminéralisée. Le chloro-1-butane, solvant d'extraction, est enfin séparé de la fraction insaponifiable résiduelle dans un évaporateur de type flot tombant. La fraction obtenue (19,3 kg) est fortement enrichie en tocophérols (45%).

Ces résultats montrent que le chloro-1-butane est un bon solvant d'extraction de la fraction insaponifiable d'un déodistillat, notamment de soja, et permet d'extraire sélectivement une des fractions actives de cet insaponifiable, tels que les stérols ou les tocophérols.

## Revendications

1. Procédé d'extraction de la fraction insaponifiable contenue dans une huile végétale ou d'un co-produit de l'industrie du raffinage des huiles végétales comprenant au moins :

    A) une étape au cours de laquelle ladite huile est transformée en une solution hydroalcoolique qui est de préférence une étape choisie dans le groupe constitué par les saponifications et les estérifications,
    B) une étape d'extraction de la solution hydro-alcoolique au cours de laquelle la fraction grasse est séparée de la fraction insaponifiable par extraction liquide-liquide,
    C) éventuellement, une étape de purification de la fraction insaponifiable choisie dans le groupe constitué par les cristallisations et les extractions liquide-liquide,

    **caractérisé en ce qu'**au moins une étape parmi les extractions liquide-liquide de l'étape B, les cristallisations de l'étape C ou les extractions liquide-liquide de l'étape C est effectuée en utilisant le chloro-1-butane.

2. Procédé d'extraction de la fraction insaponifiable contenue dans une huile végétale selon la revendication 1 comprenant au moins :

A) une étape de saponification par laquelle ladite huile est transformée en une solution hydro-alcoolique,
B) une étape d'extraction de la solution hydro-alcoolique par un solvant organique, **caractérisé en ce que** le solvant organique est le chloro-1 -butane.

3. Procédé d'extraction de la fraction insaponifiable contenue dans une huile végétale selon la revendication 2, **caractérisé en ce que** l'huile végétale est une huile d'avocat.

4. Procédé d'extraction de la fraction insaponifiable contenue dans une huile végétale selon la revendication 2, **caractérisé en ce que** l'huile végétale est une huile de soja.

5. Procédé d'extraction de la fraction insaponifiable contenue dans une huile d'avocat selon la revendication 3 **caractérisé en ce que** l'on effectue une extraction à contre-courant de la solution hydro-alcoolique au moyen de chloro-1-butane, le rapport v/v chloro-1-butane /solution hydro-alcoolique étant compris entre 0,5 et 5.

6. Procédé d'extraction de la fraction insaponifiable contenue dans une huile d'avocat selon la revendication 5, **caractérisé en ce que** le rapport v/v chloro-1-butane / solution hydro-alcoolique est compris entre 0,9 et 1,2.

7. Procédé d'extraction de la fraction insaponifiable contenue dans une huile d'avocat selon la revendication 6, **caractérisé en ce que** le rapport v/v chloro-1-butane / solution hydro-alcoolique est d'environ 1.

8. Procédé d'extraction de la fraction insaponifiable contenue dans une huile de soja selon la revendication 4 **caractérisé en ce que** l'on effectue une extraction à contre-courant de la solution hydro-alcoolique au moyen de chloro-1-butane, le rapport (volume/volume) v/v chloro-1-butane / solution hydro-alcoolique est compris entre 0,5 et 5.

9. Procédé d'extraction de la fraction insaponifiable contenue dans une huile de soja selon la revendication 8 **caractérisé en ce que** l'on effectue une extraction à contre-courant de la solution hydro-alcoolique au moyen de chloro-1-butane, le rapport (volume/volume) v/v chloro-1-butane / solution hydro-alcoolique est compris entre 0,9 et 1,5.

10. Procédé d'extraction de la fraction insaponifiable selon la revendication 9 **caractérisé en ce que** l'on effectue une extraction à contre-courant de la solution trydro-atcoolique au moyen de chloro-1-butane, le rapport (volume/volume) v/v chloro-1-butane / solution hydro-alcoolique est d'environ 1,3.

11. Procédé d'extraction de la fraction insaponifiable d'un co-produit de l'industrie du raffinage d'une huile végétale, **caractérisé en ce que** ce co-produit est un déodistillat d'une huile végétale. ledit procédé comprenant au moins :

- une étape de saponification par laquelle le déodistillat est transformée en une solution hydro-alcoolique,
- une étape d'extraction à contre-courant de la solution hydro-alcoolique au moyen de chloro-1-butane.
- une étape de cristallisation des stérols et/ou des alcools triterpéniques,
- une étape d'isolation d'un composé actif tel que les tocophérols, les tocotriénols, le squalène, les carotènes, la sésamine, la sésamoline, étape choisie dans le groupe formé par les extractions de préférence au moyen de chloro-1-butane, et les distillations.

## Claims

1. Process for extracting of the unsaponifiable fraction contained in a vegetable oil or of a coproduct of the vegetable oil refining industry, comprising at least:

A) a step during which the said oil is converted to an aqueous-alcoholic solution which is preferably a step chosen from the group consisting of saponifications and esterifications,
B) a step of extraction of the aqueous-alcoholic solution during which the fatty fraction is separated from the unsaponifiable fraction by liquid-liquid extraction,
C) optionally, a step of purification of the unsaponifiable fraction chosen from the group consisting of crystallizations and liquid-liquid extractions,

**characterized in that** at least one step among the liquid-liquid extractions of step B, the crystallizations of step C or the liquid-liquid extractions of step C is carried out using 1-chlorobutane.

2. Process for extracting the unsaponifiable fraction contained in a vegetable oil according to Claim 1, comprising at least:

   A) a saponification step by which the said oil is converted to an aqueous-alcoholic solution,
   B) a step of extraction of the aqueous-alcoholic solution with an organic solvent, **characterized in that** the organic solvent is 1-chlorobutane.

3. Process for extracting the unsaponifiable fraction contained in a vegetable oil according to Claim 2, **characterized in that** the vegetable oil is an avocado oil.

4. Process for extracting the unsaponifiable fraction contained in a vegetable oil according to Claim 2, **characterized in that** the vegetable oil is a soybean oil.

5. Process for extracting the unsaponifiable fraction contained in an avocado oil according to Claim 3, **characterized in that** a countercurrent extraction of the aqueous-alcoholic solution is carried out by means of 1-chlorobutane, the v/v 1-chlorobutane/aqueous-alcoholic solution ratio being between 0.5 and 5.

6. Process for extracting the unsaponifiable fraction contained in an avocado oil according to Claim 5, **characterized in that** the v/v 1-chlorobutane/aqueous-alcoholic solution ratio is between 0.9 and 1.2.

7. Process for extracting the unsaponifiable fraction contained in an avocado oil according to Claim 6, **characterized in that** the v/v 1-chlorobutane/aqueous-alcoholic solution ratio is about 1.

8. Process for extracting the unsaponifiable fraction contained in a soybean oil according to Claim 4, **characterized in that** a countercurrent extraction of the aqueous-alcoholic solution is carried out by means of 1-chlorobutane, the (volume/volume) v/v 1-chlorobutane/aqueous-alcoholic solution ratio being between 0.5 and 5.

9. Process for extracting the unsaponifiable fraction contained in a soybean oil according to Claim 8, **characterized in that** a countercurrent extraction of the aqueous-alcoholic solution is carried out by means of 1-chlorobutane, the (volume/volume) v/v 1-chlorobutane/aqueous-alcoholic solution ratio being between 0.9 and 1.5.

10. Process for extracting the unsaponifiable fraction according to Claim 9, **characterized in that** a countercurrent extraction of the aqueous-alcoholic solution is carried out by means of 1-chlorobutane, the (volume/volume) v/v 1-chlorobutane/aqueous-alcoholic solution ratio being about 1.3.

11. Process for extracting the unsaponifiable fraction of a coproduct of the vegetable oil refining industry, **characterized in that** this coproduct is a deodistillate of a vegetable oil, the said process comprising at least:

   - a saponification step by which the deodistillate is converted to an aqueous-alcoholic solution,
   - a step of countercurrent extraction of the aqueous-alcoholic solution by means of 1-chlorobutane,
   - a step of crystallization of the sterols and/or of the triterpenic alcohols,
   - a step of isolation of an active compound such as tocopherols, tocotrienols, squalene, carotenes, sesamin or sesamolin, which step is chosen from the group consisting of extractions, preferably by means of 1-chlorobutane, and distillations.

## Patentansprüche

1. Verfahren zur Extraktion der unverseifbaren Fraktion, die in einem Pflanzenöl oder in einem Nebenprodukt der Industrie der Raffination von Pflanzenölen enthalten ist, welches mindestens umfasst:

   A) einen Schritt, bei dem das Öl zu einer wässrig-alkoholischen Lösung verarbeitet wird, welcher vorzugsweise ein Schritt ist, der aus der Gruppe ausgewählt ist, die aus Verseifungen und Veresterungen besteht,
   B) einen Schritt der Extraktion der wässrig-alkoholischen Lösung, bei dem die Fettfraktion durch Flüssig-Flüssig-Extraktion von der unverseifbaren Fraktion abgetrennt wird,

C) gegebenenfalls einen Schritt der Reinigung der unverseifbaren Fraktion, der aus der Gruppe ausgewählt ist, die aus Kristallisationen und Flüssig-Flüssig-Extraktionen besteht,

**dadurch gekennzeichnet, dass** mindestens ein Schritt aus den Flüssig-Flüssig-Extraktionen des Schrittes B, der Kristallisationen des Schrittes C oder der Flüssig-Flüssig-Extraktionen des Schrittes C unter Verwendung von 1-Chlorbutan bewirkt wird.

**2.** Verfahren zur Extraktion der unverseifbaren Fraktion, die in einem Pflanzenöl enthalten ist, nach Anspruch 1, welches mindestens umfasst:

A) einen Schritt der Verseifung, durch welchen das Öl zu einer wässrig-alkoholischen Lösung verarbeitet wird,
B) einen Schritt der Extraktion der wässrig-alkoholischen Lösung durch ein organisches Lösungsmittel, **dadurch gekennzeichnet, dass** das organische Lösungsmittel 1-Chlorbutan ist.

**3.** Verfahren zur Extraktion der unverseifbaren Fraktion, die in einem Pflanzenöl enthalten ist, nach Anspruch 2, **dadurch gekennzeichnet, dass** das Pflanzenöl ein Avocadoöl ist.

**4.** Verfahren zur Extraktion der unverseifbaren Fraktion, die in einem Pflanzenöl enthalten ist, nach Anspruch 2, **dadurch gekennzeichnet, dass** das Pflanzenöl ein Sojaöl ist.

**5.** Verfahren zur Extraktion der unverseifbaren Fraktion, die in einem Avocadoöl enthalten ist, nach Anspruch 3, **dadurch gekennzeichnet, dass** man eine Gegenstromextraktion der wässrig-alkoholischen Lösung mittels 1-Chlorbutan bewirkt, wobei das Verhältnis Vol./Vol. 1-Chlorbutan/wässrigalkoholische Lösung zwischen 0,5 und 5 eingeschlossen beträgt.

**6.** Verfahren zur Extraktion der unverseifbaren Fraktion, die in einem Avocadoöl enthalten ist, nach Anspruch 5, **dadurch gekennzeichnet, dass** das Verhältnis Vol./Vol. 1-Chlorbutan/wässrig-alkoholische Lösung zwischen 0,9 und 1,2 eingeschlossen beträgt.

**7.** Verfahren zur Extraktion der unverseifbaren Fraktion, die in einem Avocadoöl enthalten ist, nach Anspruch 6, **dadurch gekennzeichnet, dass** das Verhältnis Vol./Vol. 1-Chlorbutan/wässrig-alkoholische Lösung etwa 1 beträgt.

**8.** Verfahren zur Extraktion der unverseifbaren Fraktion, die in einem Sojaöl enthalten ist, nach Anspruch 4, **dadurch gekennzeichnet, dass** man eine Gegenstromextraktion der wässrig-alkoholischen Lösung mittels 1-Chlorbutan bewirkt, das Verhältnis (Volumen/Volumen) Vol./Vol. 1-Chlorbutan/wässrig-alkoholische Lösung zwischen 0,5 und 5 eingeschlossen beträgt.

**9.** Verfahren zur Extraktion der unverseifbaren Fraktion, die in einem Sojaöl enthalten ist, nach Anspruch 8, **dadurch gekennzeichnet, dass** man eine Gegenstromextraktion der wässrig-alkoholischen Lösung mittels 1-Chlorbutan bewirkt, das Verhältnis (Volumen/Volumen) Vol./Vol. 1-Chlorbutan/wässrig-alkoholische Lösung zwischen 0,9 und 1,5 eingeschlossen beträgt.

**10.** Verfahren zur Extraktion der unverseifbaren Fraktion nach Anspruch 9, **dadurch gekennzeichnet, dass** man eine Gegenstromextraktion der wässrig-alkoholischen Lösung mittels 1-Chlorbutan bewirkt, wobei das Verhältnis (Volumen/Volumen) Vol./Vol. 1-Chlorbutan/wässrig-alkoholische Lösung etwa 1,3 beträgt.

**11.** Verfahren zur Extraktion der unverseifbaren Fraktion eines Nebenprodukts der Industrie der Raffination eines Pflanzenöls, **dadurch gekennzeichnet, dass** das Nebenprodukt ein Deodestillat eines Pflanzenöls ist,
wobei das Verfahren mindestens umfasst:

- einen Schritt der Verseifung, durch welchen das Deodestillat zu einer wässrig-alkoholischen Lösung verarbeitet wird,
- einen Schritt der Gegenstromextraktion der wässrig-alkoholischen Lösung mittels 1-Chlorbutan,
- einen Schritt der Kristallisation von Sterolen und/oder Triterpenalkoholen,
- einen Schritt der Isolation einer aktiven Verbindung, wie Tocopherole, Tocotrienole, Squalen, Carotine, Sesamin, Sesamolin, wobei der Schritt aus der Gruppe ausgewählt ist, die aus den Extraktionen vorzugsweise mittels 1-Chlorbutan und Destillationen besteht.